# EUROPEAN PATENT SPECIFICATION

(11) **EP 4 110 235 B1**
(45) Date of publication and mention of the grant of the patent: **28.01.2026**
(21) Application number: 21761245.6
(22) Date of filing: 27.02.2021
(51) Int. Cl.: A61F 2/38, A61F 2/00, A61F 2/02, A61F 2/08, A61F 2/30

(54) **IMPLANTABLE KNEE PROSTHESIS WITH INTEGRATED PROSTHETIC EXTENSOR MECHANISM**
IMPLANTIERBARE KNIEPROTHESE MIT INTEGRIERTEM PROTHESENAUSSTRECKUNGSMECHANISMUS
PROTHÈSE DE GENOU IMPLANTABLE AVEC MÉCANISME EXTENSEUR PROTHÉTIQUE INTÉGRÉ

(30) Priority: 28.02.2020 US 202062983560 P; 08.07.2020 US 202063049612 P
(43) Date of publication of application: 04.01.2023
(73) Proprietor: Extensor, LLC, Houston, TX 77019 (US)
(72) Inventor: STOCKS, Gregory, W., Houston, TX 77040 (US); BRINKER, Mark, R., Houston, TX 77040 (US)
(74) Representative: Vesterinen, Jussi Tapio
(86) International application number: PCT/US2021/020140
(87) International publication number: WO 2021/174156

(56) References cited:
- US-A- 3 848 276
- US-A- 3 909 854
- US-A- 3 990 116
- US-A- 4 204 284
- US-A1- 2002 120 340
- US-A1- 2011 060 422
- US-A1- 2011 106 265
- US-A1- 2014 350 674

## Description

### BACKGROUND OF THE INVENTION

The present inventions relate to an implantable total knee prosthetic for patients with compromised or non-existing extensor systems.

### Description of the Related Art.

Conventional knee replacements, e.g., total knee arthroplasty, require a viable extensor system, which for purposes of this disclosures refers to the muscles, tendons, ligaments, and kneecap that provide stability to the knee joint and that allows a person to straighten her knee. If a person requires a total knee replacement, but does not have a functional extensor system, the most common options are amputation above the knee with either osteointegration or socket technology with and external knee prosthetic, or total fusion of the knee joint (*in situ* or *ex situ*). Both procedures may lead to a reduction in the quality of life. However, over 150,000 leg amputations occur annually in the United States. While the above-the-knee (transfemoral) amputation occurs less often than the below-the-knee (transtibial) amputation, above-the-knee amputation results in the highest level of dysfunction and disability.

It is known that the design and configuration of the knee prosthesis dictates the degree of voluntary control required by the amputee in the various phases of the walking cycle. The main requirement in the replacement of the knee joint is to design a device that permits the amputee to walk comfortably and safely without undue mental or physical effort. During a single gait cycle, each leg passes through an extension phase and one swing phase. The extension phase begins when the heel touches (or strikes) the floor. Thereafter, the sole of the foot contacts the ground. Next, the body weight is swung directly over the supporting leg and continues to rotate over the foot (mid-stance). As the body mass above the ankle continues to rotate forward, the heel lifts off the ground. After heel lifts off, the body is moved forward by the calf muscles. The cycle ends when the entire foot rises from the ground. In level walking the normal human knee rotates through a range of approximately 70° going from a position of full extension (0°) in early and mid-stance to 70° of flexion shortly after toe-off. A prosthetic knee should allow flexion while compensating for the loss of muscle control both regarding stability and swing control. Therefore, in designing a prosthetic knee joint, it is desirable to approximate the position and motion of the natural knee joint as closely as possible.

The knee joint comprises two basic movements, flexion and extension in the sagittal plane, and internal and external rotation about a vertical axis. The cartilage, menisci, ligaments, and muscles around the knee react substantial stresses during daily activities. If the intrinsic soft tissue stability of the knee is compromised or non-existent, laxity in flexion and extension increases and may result in a flexion gap imbalance. Thus, a prosthesis should approximate as closely as possible the joint laxity and stability of a normal knee.

To minimize or prevent uncontrolled movement during the swinging phase of an external knee prosthesis, a braking device, such as an adjustable braking device, may be provided to bias, regulate, or dampen, to a degree, the action of the forces of inertia during the swing phase. For example, a brake may continuously engage and disengage during the swing-phase of ambulation.
US3990116 discloses an improved pretensioned prosthetic device, which incorporates pretensioned or biased spring means to provide a skeletal joint such as a finger or knee joint with a controlled flex and reflex action. US4204284 discloses a joint prosthesis comprising two relatively inextensible primary components that are spaced apart from one another. Disposed between and spaced from each of the primary components is a pivot member that is also fabricated of relatively inextensible material. US3848276 discloses a joint prosthesis particularly useful in total knee replacement, and capable of providing controlled motion about three axes corresponding to the capabilities of a normal knee. A dual centered pivot with contacting arcuate support blocks provides for a combination of rotational and translational movement during knee bending movements.

The present invention is defined in claim 1 and is directed to an implantable total knee prosthetic for use with patients that would otherwise require above-the-knee amputation or total knee fusion.

### BRIEF DESCRIPTION OF THE SEVERAL VIEWS OF THE DRAWINGS

The following figures form part of the present specification and are included to demonstrate further certain aspects of the present invention. Our inventions may be better understood by reference to one or more of these figures in combination with the detailed description of specific embodiments presented herein. However, neither the detailed description nor the figures are intended or provided to limit the scope of the claims.
FIG. 1 illustrates a typical human knee.
FIG. 2 illustrates the extensor mechanism of a typical human knee.
FIG. 3 illustrates an *in situ* total knee prosthesis having an integrated prosthetic extensor mechanism for persons with compromised extensor mechanisms.
FIG. 4 illustrates an *in situ* intermedullary knee prosthesis having an integral extensor mechanism for persons with compromised extensor systems.
FIG. 5 illustrates another embodiment of an *in situ* total knee prosthesis having an integrated extensor mechanism for persons with compromised extensor systems.
FIG. 6A illustrates an embodiment of a smart prosthetic knee and associated technology.
FIG. 6B illustrates an embodiment of a control system for smart prosthesis.
FIG. 7 illustrates a logic flow chart for a top level algorithm software useful with embodiments of the present inventions.
FIG. 8 illustrates an embodiment of a rotation mechanism for an in *situ* total knee prosthesis having an integrated extensor mechanism for persons with compromised extensor systems.
FIG. 9 illustrates another embodiment of an *in situ* total knee prosthesis having an integrated extensor mechanism for persons with compromised extensor systems.

While the inventions disclosed herein are susceptible to various modifications and alternative forms, only a few specific embodiments have been shown by way of example in the drawings and are described in detail below. The figures and detailed descriptions of these specific embodiments are not intended to limit the breadth or scope of the inventive concepts or the appended claims in any manner. Rather, the figures and detailed written descriptions are provided to illustrate the inventive concepts to a person of ordinary skill in the art and to enable such person to make and use the inventive concepts.

### DETAILED DESCRIPTION

The Figures described above, and the written description of specific structures and functions below are not presented to limit the scope of what we have invented or the scope of the issued claims. Rather, the Figures and written description are provided to teach any person skilled in the art to make and use the inventions for which patent protection is sought. Those skilled in the art will appreciate that not all features of a commercial embodiment of the inventions are described or shown for the sake of clarity and understanding. Persons of skill in this art will also appreciate that the development of an actual commercial embodiment incorporating aspects of the present inventions will require numerous implementation-specific decisions to achieve the developer's ultimate goal for the commercial embodiment. Such implementation-specific decisions may include, and likely are not limited to, compliance with system-related, business-related, government-related, and other constraints, which may vary by specific implementation, location and from time to time. While a developer's efforts might be complex and time-consuming in an absolute sense, such efforts would be, nevertheless, a routine undertaking for those of skill in this art having benefit of this disclosure. It must be understood that the inventions disclosed and taught herein are susceptible to numerous and various modifications and alternative forms. Lastly, the use of a singular term, such as, but not limited to, "a," is not intended as limiting of the number of items. Also, the use of relational terms, such as, but not limited to, "top," "bottom," "left," "right," "upper," "lower," "down," "up," "side," and the like are used in the written description for clarity in specific reference to the Figures and are not intended to limit the scope of the invention or the appended claims.

Aspects of the inventions disclosed herein may be embodied as an apparatus or system. Accordingly, specific embodiments may take the form of an entirely hardware embodiment or an embodiment combining software and hardware aspects, such as a "circuit," "module" or "system." Furthermore, embodiments of the present inventions may involve a computer program product embodied in one or more computer readable storage media having computer readable program code.

Items, components, functions, or structures in this disclosure may be described or labeled as a "module" or "modules." For example, but not limitation, a module may be configured as a hardware circuit comprising custom VLSI circuits or gate arrays, off-the-shelf semiconductors such as logic chips, transistors, or other discrete components. A module also may be implemented as programmable hardware devices such as field programmable gate arrays, programmable array logic, programmable logic devices, or the like. Modules also may be configured as software for execution by various types of processors. A module of executable code may comprise one or more physical or logical blocks of computer instructions that may be organized as an object, procedure, or function. The executables of a module need not be physically located together but may comprise disparate instructions stored in different locations that when joined logically together, comprise the module and achieve the stated purpose or function. A module of executable code may be a single instruction, or many instructions, and may even be distributed over several different code segments, among different programs, and across several memory devices. Similarly, data may be identified and illustrated herein within modules, and may be embodied in any suitable form and organized within any suitable type of data structure. The data may be collected as a single dataset or may be distributed over different locations including over different storage devices, and may exist, at least partially, merely as electronic signals on a system or network. Where a module or portions of a module are implemented in software, the software portions may be stored on one or more computer readable storage media.

Reference throughout this disclosure to "one embodiment," "an embodiment," or similar language means that a particular feature, structure, or characteristic described in connection with the embodiment is included in at least one of the many possible embodiments of the present inventions. The terms "including," "comprising," "having," and variations thereof mean "including but not limited to" unless expressly specified otherwise. An enumerated listing of items does not imply that any or all the items are mutually exclusive and/or mutually inclusive, unless expressly specified otherwise. The terms "a," "an," and "the" also refer to "one or more" unless expressly specified otherwise.

Furthermore, a described feature, structure, or characteristic of one embodiment may be combined in any suitable manner in one or more other embodiments. In the following description, numerous specific details are provided, such as examples of programming, software modules, user selections, network transactions, database queries, database structures, hardware modules, hardware circuits, hardware chips, etc., to provide a thorough understanding of embodiments of the disclosure. Those of skill in the art having the benefit of this disclosure will understand that the inventions may be practiced without one or more of the specific details, or with other methods, components, materials, and so forth. In other instances, well-known structures, materials, or operations are not shown or described in detail to avoid obscuring aspects of the disclosure.

The description of elements in each Figure may refer to elements of proceeding Figures. Like numbers refer to like elements in all figures, including alternate embodiments of like elements. In some possible embodiments, the functions/actions/structures noted in the figures may occur out of the order noted in the block diagrams and/or operational illustrations. For example, two operations shown as occurring in succession, in fact, may be executed substantially concurrently or the operations may be executed in the reverse order, depending upon the functionality/acts/structure involved.

In general, our inventions relate to an implantable total knee prosthesis for individuals that have compromised or non-functional extensor systems. The prostheses of the present inventions comprise an integrated braking mechanism configured to provide a constant force to the joint to simulate or approximate a level of normal knee function. Above the knee amputees with socket prostheses or osseointegration prostheses may have additional mechanical, hydraulic, or electronic braking and/or extension devices to compensate for the non-functional or non-existent extensor systems. It will be appreciated that individuals having a conventional knee replacement with a compromised extensor system cannot walk without the fear of falling and cannot climb steps or stairs. In contrast, our implantable prosthetic knee joints with an integral or integrated extensor mechanism may improve a person's ability to walk and/or even climb stairs without fear of the knee buckling, and thereby avoid the requirement of above the knee amputation or join fusion.

It will be appreciated that the inventions are disclosed with respect to the knee joint, our inventions may be applicable to other joints, such as, for example, ankle joints.

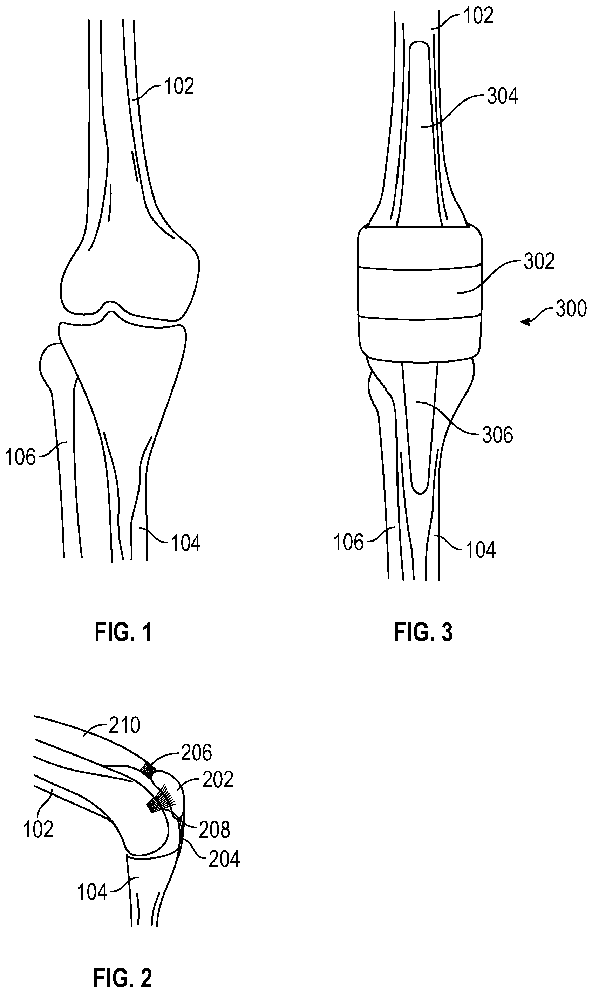

FIGs. 1 and 2 illustrate components of a typical human knee joint. These figures illustrate the femur 102, the tibia 104, the fibula 106, and the patella 202. FIG. 2 illustrates the patellar ligament 204, the quadriceps tendon 206, and the patellar retinaculum 208. It is understood that the extensor mechanism of the knee consists of the quadriceps muscles 210, quadriceps tendon 206, patella 202, patellar ligament 204, patellar retinaculum (medial and lateral) and adjacent soft tissues.

FIG 3 illustrates an implanted total prosthetic knee system 300 having a knee joint 302 with an integrated prosthetic extensor mechanism according to the teachings of the present invention. The prosthesis system 300 replaces the end of the femur 102 or top of the tibia 104 or both, such as when the bone 102, 104 has been severely damaged by infection, fracture, or tumor, e.g., a tumor total knee arthroplasty. The integrated prosthetic extensor mechanism (not shown) allows an individual with a compromised or non-functional extensor mechanism to avoid amputation or fusion of the knee joint and increase the ability to walk and/or even climb or descend steps and stairs.

As described below, a prosthetic extensor mechanism useful with all embodiments of the disclosed embodiments comprises a biasing mechanism configured to exert a constant, varying, and/or adjustable force to one or more components of the prosthetic joint 302 to bias the prosthetic system 300 and therefore the leg to the extended position. Suitable biasing mechanisms include spiral springs, wafer springs, hydraulic cylinders, pneumatic cylinders, magnets, and linear motors.

addition, the prosthetic extensor mechanism includes a brake or resistance mechanism configured to apply constant pressure against a rotating surface to slow or retard the rate or range of movement (flexion) of the joint 302. Suitable resistance mechanisms include camming surface, interference fit surfaces, magnets, spiral springs, wafer springs, hydraulic cylinders, pneumatic cylinders, magnets, and linear motors. It will be appreciated a biasing mechanism also can function as a resistance mechanism, and *vice versa.*

In the illustration of the prosthetic knee system 300, the system 300 has an integral or modular intermedullary femoral stem 304, and an integral or modular intermedullary tibial stem 306. In prosthetic systems that have integral stems, implantation may require dissembling the joint 302. Alternately, the during implantation, the human knee joint may be hyper-extended, and the complete prosthetic system 300 implanted with integral stems and without disassembling the joint 302. In prosthetic systems 300, having one or more modular stems, the stems may be implanted and then the joint 302 coupled to the one or more modular stems 304, 306.

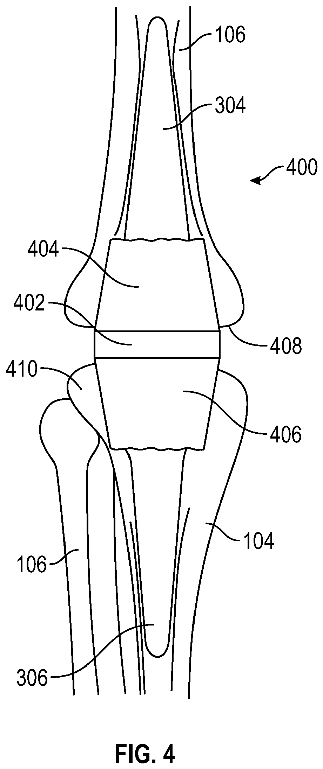

FIG. 4 illustrates another embodiment of the invention that does not require substantial removal (e.g., about 5+ cm) of bone from the femur 102 or tibia 104 and therefore could be used for individuals that have not lost significant amount of bone due to severe irreparable fracture, infection, or tumor. Instead, most of the prosthetic system 400 illustrated in FIG. 4 is encased within the bone of the end of the femur 408 and/or top of the tibia 410. As illustrated, the femoral component 404 of the prosthetic joint 402 is substantially encased in the epicondyle, and the tibial component 406 of the prosthetic joint 402 is substantially encased in the epicondyle.

The joints 302, 402 suitable for use with the present inventions include, but are not limited to, monocentric (single) axis joints that operate as a simple hinge; polycentric (multi) axis joint that have multiple pivot points; a manual friction joint that uses a "brake" to vary the resistance to flexion; a variable friction joint that utilizes a fluid control system to control resistance to knee bending; a pneumatic fluid control joint to vary the resistance to flexion; a hydraulic fluid control joint configured to control knee speed; a locking joint that can be locked to simulate a fused knee joint; a weight-activated stance-control joint; and a computerized or "smart" joint.

Those of skill having benefit of this disclosure will appreciate that there are on the market prosthetic knee systems configured for *ex situ* use (*i.e*., not implanted) that may be modified for use as an implantable joint. For example, and not limitation, the Ottobock company manufactures above the knee prostheses, including a microprocessor-based system known as the C-Leg. One or more of these external prostheses may form the basic joint similar to that described herein. Persons of skill will appreciate that there are other external prosthetic knees suitable for use in similar applications.

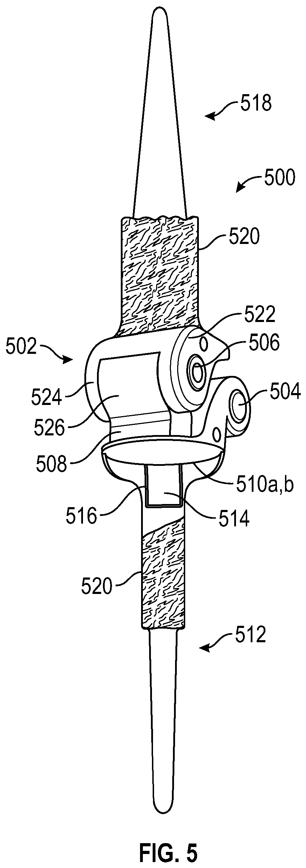

FIG. 5 illustrates another of many possible embodiments of an *in situ* prosthetic knee system 500 according to the present inventions. Knee joint 502 represents, without limitation, a modified polycentric *ex situ* knee joint having two offset hinge points 504 and 506, and an impact pad 508. As is known, the pad 508 may be fabricated from a bio-compatible wear-resistant material, such as ultrahigh molecular weight polyethylene (UHWMPE). While a polycentric *ex situ* total knee joint is illustrated, it will be appreciated that most any *ex situ* total replacement knee joint may be used with the teachings of these inventions.

The knee joint 502 preferably comprises a rotation system configured to permit some amount of rotation about an axis substantially normal to the hinge axis 504 or 506, or substantially parallel to a vertical axis. For example, and not limitation, knee joint 502 may comprise rotation surfaces 510a and 510b, such as spherical mating surfaces, that allow relative rotation between the knee joint 502 and, for example, the tibial stem 512. It is preferred that either or both of surfaces 510a or 510b comprise a wear resistant surface with sufficient lubricity to allow a normal feel of rotation under load, such as UHMWPE. While the point or area of rotation is preferably associated with the knee joint 502 below the impact pad 508, it will be appreciated that the point of rotation may be associated with the knee joint 502 at or above the hinge points 504 or 506.

The rotation system preferably comprises one or more structures, such as, but not limited to, stops, configured to limit the amount of relative rotation. For example, the knee joint 502 illustrated in FIG. 5 comprises a projection 514 extending downwardly into the tibial stem 512. The projection 514 may comprise one or more ears, lugs, or recesses 516 that cooperate with the tibial stem 512 to limit the amount of rotation, such as, to about ±15 degrees. The structures that may be provided to limit an amount or type of movement may be configured to be generally applicable to most patients. As a non-limiting example, a tibial rotation within our implantable prosthetic knee system may be configured to limit that movement to a norm expected by the 95th percentile of healthy adults. Other *in situ* rotation systems are contemplated for the inventions disclosed herein. In addition, *ex situ* rotating hinge knee protheses may be utilized with the present inventions. In some embodiments, the resistance to rotation may increase as the amount of rotation increases. Camming surfaces or interference surface may be employed to create a varying amount of resistance.

As disclosed elsewhere in this specification, these issues of rotation may be addressed adjusting the structures that provide stops, limits, and biases pre-implantation or post-implantation. Alternatively, these issues may be proactively addressed by assessing the current mobility and extensor ranges for candidates for joint replacement. The measured ranges and limits may be configured by making initial adjustments or by manufacturing to specified tolerances and/or functionality.

Applicants envision further embodiments that make use of the features of a "smart" or microprocessor-based prosthetic system. In one of many possible embodiments that may be envisioned by those having benefit of this disclosure, a "smart" joint may be configured to communicate with wearable sensors, such as accelerometers, position sensors, angle sensors, pressure sensors, and/or load sensors to self-adjust ranges, limits and/or functions of the implanted prosthetic joint (or joints) for a particular person and/or for particular situations.

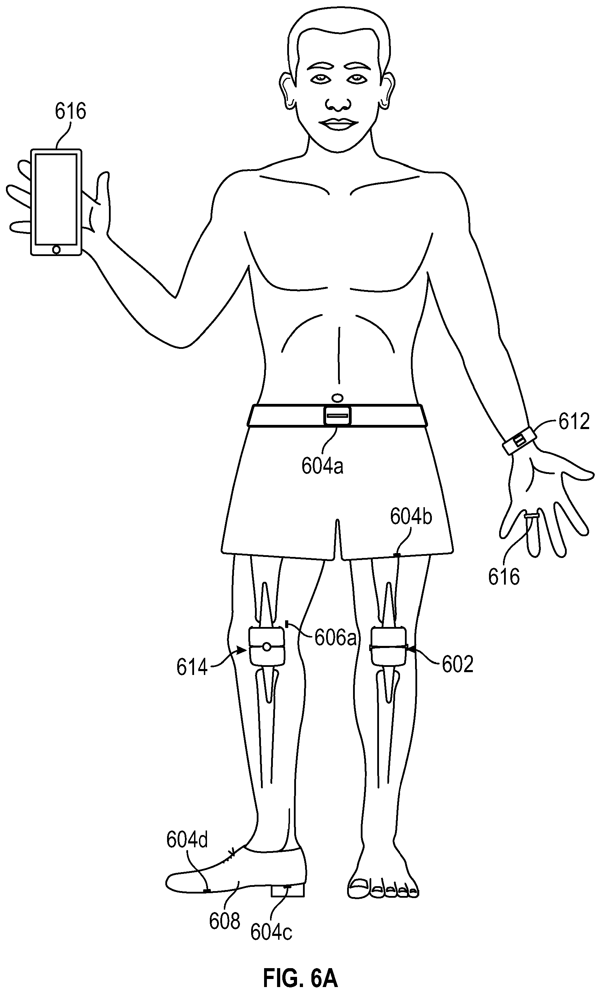

In one non-limiting embodiment illustrated in FIG. 6A, a "smart" prosthetic knee system 602 may receive inputs from wearable sensors, such as one or more sensors in a person's belt 604a, clothing 604b, shoes 604c, 604d or the like to set ranges and limitations of the system 602. These articles of clothing have the advantage that they are normally worn at specific locations relative to the knee and for specific activities. Placing sensors in belts, shoes and/or clothing is also much less encumbering than having to wear a sensor that would have to be applied at a specific location on a body, such as on the contralateral knee. However, the inventions disclosed herein may make use of sensors removably or permanently attached to the body.

In this envisioned embodiment, sensors in the belt 604a may relay pressure changes such as may be a contraction of a dorsi or gluteal muscle that may be a precursor to a push off step if the shoe sensor identifies that the legs are fully extended - *e.g.,* the ipsilateral shoe is at a maximum distance below the "smart" knee 602 and the contralateral shoe is appropriately nearby as would be normal for a stance. A small pressure change detected along the belt may indicate that the wearer is preparing to take a small or normal step. However, a larger pressure change detected may indicate that the wearer is preparing for a leap or lunge. The "smart" knee 602 may then process these signals and make instantaneous adjustments to its limits and ranges in anticipation of this activity.

In another embodiment, the collection of sensors may indicate that the shoes 604c, 604d are nearing the belt 604a. If the belt 604a is substantially vertically above the shoes, the "smart" knee 602 may anticipate that the wearer is crouching or perhaps preparing to sit. However, if the belt is offset from the shoes, it may be anticipated that the wearer is preparing to kneel. Algorithms may be implemented in the prosthetic knee 602 to temporarily adjust an amount of friction, resistance, or bias to the anticipated movement. Appropriate adjustments may be made to the ranges, limits, and even to the biasing of the "smart" knee for these interpreted sensor inputs.

In yet another envisioned embodiment, the pressures of the fronts and backs of the soles of the feet 604c, 604d in relationship to the distance between the feet may indicate that the wearer is walking or running. Greater pressure differences and longer strides may indicate running, which may require a larger extension bias than a normal walking stride. Similarly, sensing that one shoe has no sole pressure 604d while also sensing a rise in elevation may indicate the need to make adjustments for the wearer to walk up an incline or even steps.

While the examples given here are for sensors in a belt and shoes in relation to the prosthetic knee, Applicants envision embodiments with other sensors worn or placed on the body and used in relation to other implantable extensor joints. Without limitation, these may include appropriate sensors in wrist watches 612, rings 616, bracelets 612, necklaces, piercings, and any other device that may be worn on the body. Sensors that rely on a distance from a "smart" extensor joint should maintain a relatively stable position relative to the body or relative to the joint or joints they support. Embodiments of inventions described herein may include sensors that monitor and relay other information such as, but not limited to, temperature, direction of gaze, respiration, heartbeat (all of which may be used as indicators of upcoming movements requiring joint extensions).

"Smart" joints 602 may communicate with nearby sensors through means known to those of ordinary skill in the art. These communications pathways may include, without limit, near field communication (NFC) networks, Bluetooth including Bluetooth low energy (BLE), and several other radio frequency pathways.

Applicants envision that some people may have multiple "smart" joints 602, 614 and that it may be beneficial for the "smart" joints to communicate with each other. As an example, but without limitation, a first "smart" knee 502 may work in tandem with a second smart knee 614. One or more sensors embedded in the first and second knee prosthetic may allow each smart knee to monitor the activity (including inactivity) of the other knee to for use by the movement algorithm in creating the desired amount of functionality (e.g., resistance to flexion). Alternately, external sensor may be worn on clothing, such as pants, adjacent each knee.

As another example, but without limitation, a first "smart" knee 602 may work in tandem with an ipsilateral "smart" ankle (not shown). In one of many envisioned embodiments, the "smart" joints may coordinate their activities. These activities may include, without limitation, self-adjustments to replicate normal and/or expected movements such as walking, climbing, reclining, stretching, and all other movements that people may perform. A practical example would be setting appropriate biases, motion ranges, and extensions in the "smart" joints when climbing stairs. In this, the "smart" ankle may aid the inclination of the front of the foot at the time that the "smart" knee moves upwards.

The "smart" joints may communicate with each other in ways that are similar to how they would communicate with sensors. However, this may entail propagating radio frequency signals from a transmitter inside the body to a receiver that is also inside the body, which may not be efficient. Applicants envision that in addition to the methods described within this specification and others that would be known to those ordinarily skilled in the appropriate art, other means of communication may be deployed. Some examples, without limitation, may include electrically conductive media, such as a wire or wires, that may be subcutaneously inserted to facilitate these communications.

These "smart" joints may also receive configuration commands from external devices and may transmit data to external devices. In one of many non-limiting envisioned embodiments, a person with a "smart" knee may link the "smart" knee to their cell phone 616 or other similar device using known wireless communication and/or telemetry protocols. The cell phone 616 or other device may make use of application software designed and configured to interface with the smart knee. For example, before a patient begins a jog or brisk walk, the patient may "program" a smart knee to a desired level of functionality. Similarly, while jogging or walking, the patient may program or reprogram the smart knee as desired.

As another example, a "smart" knee application may work in tandem with a map feature to geographically locate the person and anticipate their direction and speed and send configuration commands to the "smart" knee. For example, if the map feature indicates that the person is approaching a steep incline, the cell phone may instruct the "smart" knee to set ranges, limits, and biases to appropriate settings for climbing. The ranges, limits, and biases may be kept there until the map feature indicates that the person has moved to different terrain.

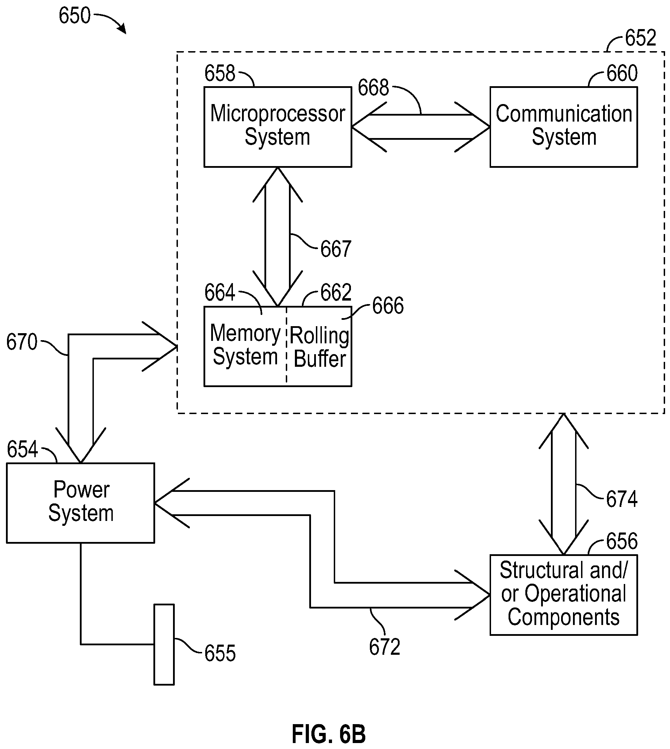

FIG. 6B illustrates an example of a control system 650 comprising a controller 652, a power system 654, the structural and or operational components 656 in the prosthesis that can be adjusted or controlled, such as, without limitation, a prosthetic extensor mechanism and/or a prosthetic resistance mechanism, or both. The controller 652 preferably comprises a microprocessor subsystem 658 configured to execute algorithms (software), firmware and other logic control instructions. The microprocessor subsystem 658 is configured communicate with a communication subsystem 660, such as over bus 668. Communication subsystem 660 is configured to receive data from one or more internal or external sensors, as described above, and to communicate such data to the microprocessor subsystem 658 over bus 668. The communication system 660 also may be configured to communicate with external smart devices running dedicated application software, as described above. It will be appreciated that the communication protocol implemented and executed by the control system 652 preferably will be a combination of wired and wireless protocols. For example, and not limitation, sensor internal to the prosthesis may be wired to the communication subsystem 660, and sensors external to the prosthesis, such as, but not limited to, belt sensor 604a, may communicate wirelessly.

The controller 652 may comprise a memory subsystem 662 configured to store 664 the prosthetic control and learning algorithms, firmware, and other algorithms, as well as store sensor data and software data, such as generated control instructions. The memory subsystem 662 may preferably comprise a rolling buffer 666 configured to receive the raw sensor data. The algorithms executed by the microprocessor 658 may sample the sensor data in the rolling buffer 666 at periodic intervals. A communication bus 667 transfers data at least between microprocessor 658 and memory subsystem 662.

It is preferred that the controller 652 and power subsystem 654 are integrally associated with the prosthetic body or envelope that is implanted in the human knee space. For example, the controller 652 and power subsystem 654 may be packaged to reside in a portion of the femoral or tibial stem, or in the prosthetic joint between the stems. Alternately, the control controller 652 and/or power subsystem 654 may be implanted in locations in the body separated from the prosthesis and be operationally connected to the prosthetic knee 656 through wired solutions. For example, and not limitation, the power subsystem 654 may be in an area of the human body that allows better access for power subsystem 654 replacement or recharging. Further still, the power subsystem 654 may comprise a transdermal access port 655 configured to permit recharging of the power subsystem using a smart needle or other such device. Additionally, the transdermal port 655 also may allow transdermal data transfer to the controller 602.

Regardless of where the controller 652 and power subsystems 654 are located, the controller 652 and power subsystem 654 may be configured to communicate data, power, and power and data to one or more of the prosthetic extensor mechanism 656 and prosthetic resistance mechanism 656.

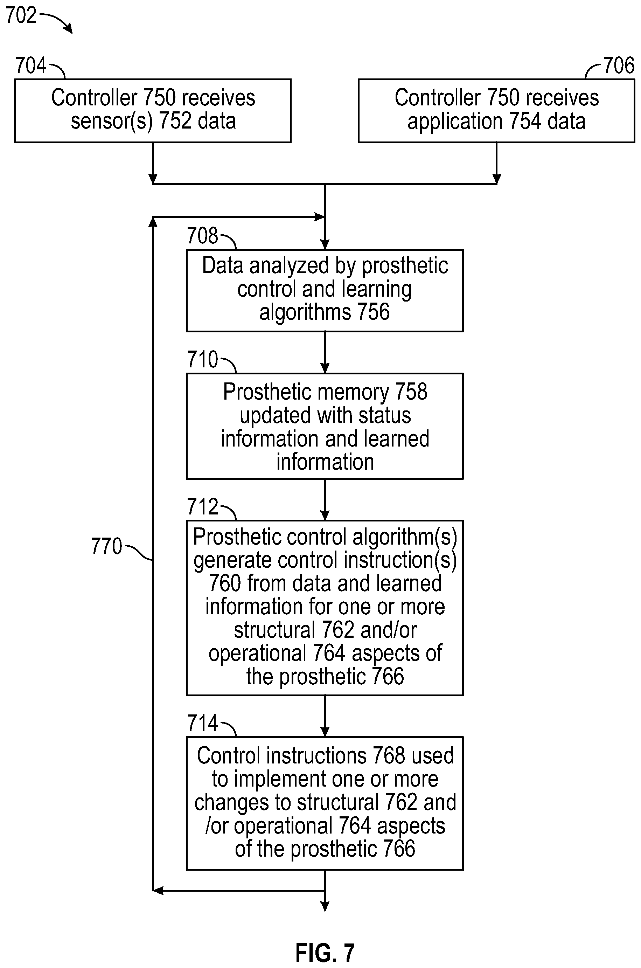

FIG. 7 illustrate one of many algorithms or software useful with embodiments of the present inventions. With non-limiting reference to the embodiment described in FIGs. 6A and 6B, flow chart 702 illustrates the logical progression of a top level algorithm for a smart prosthetic knee. The smart knee, such as 602, 614, comprises a microprocessor or controller 750 configured, such as through software or firmware, to receive information or data from one or more sensors 752. As described previously, these sensor(s) 752 may be external to the prosthetic 766, as such as, but not limited to, a belt sensor 604a, and/or may be an internal sensor associated with the prosthetic, such as, but limited to, an angular position sensor, or an accelerometer. As illustrated at step 704, the controller 750 receives data from one or more of the sensors 752.

As illustrated in step 706, the controller 750 also may be configured, such as through firmware or software, to receive data from an external smart device running a dedicated software application associated with the prosthetic 766. For example, and not limitation, the external smart device may comprise smart phone 616.

As illustrated in step 708, the data, whether sensor data, application data or both, may be analyzed or processed by prosthetic control algorithm 756, which algorithms may or may not comprise machine learning or artificial intelligence learning capabilities. It will be understood by those of skill having benefit of this disclosure that prosthetic control algorithms 756 may be configured and implemented to adjust, vary or control one or more structural or operational characteristics or functions of the prosthetic knee.

In step 710, the memory system 758 of controller 750 may be updated with some or all of the sensor 752 data and/or with status information generated by the prosthetic control algorithms and/or the learning algorithms.

In step 712, the prosthetic control algorithms 756 may generate one or more control instructions 760 from the sensor data 752, application data 754, and/or learned data. The one or more control instructions 760 may be configured to be received by the one or more adjustable or controllable structures or features of the prosthesis, such as, but not limited, the prosthetic extensor mechanism and/or the prosthetic resistance mechanism.

In step 714, the one or more control instructions 768 are used to adjust, vary or control one or more structural 762 or operational 764 characteristics or functions of the prosthetic knee 766.

The algorithm may be configured to loop 770 back to step 708 to analyze new (e.g., new in time) data from the various data sources to determine whether further changes to structural 762 and/or operational 764 characteristics of the prosthesis are needed or desired. For example, and not limitation, if one or more sensor data indicates a period of inactivity over a predetermined period, the algorithms 756 may conclude that the patient is asleep or resting, and the prosthesis and controller 750 may enter a sleep or inactivity mode to conserve power resources.

Returning to a description of other embodiments of the present invention, the *in situ* knee joint of FIG. 5 illustrates a femoral stem 518 and a tibial stem 512. Either or both of the stems 512, 518 may be fabricated integrally with the associated knee joint 502 components or may be separate or modular components. For integral stems, the knee joint 502 (that is, stems) may be implanted with one or both of hinge points 504, 506 unassembled, and then the knee joint 502 assembled after the stems are set. Alternately, for integral stems, the human knee joint may be hyper-extended to accommodate implantation of the prosthetic system. It is also contemplated that one stem may be integral with the knee joint and the other stem is modular or separable. Stems 512 and 518, whether modular or integral or a combination, may be provided in a variety of lengths and forms for a variety of potential patients For example, and not limitation, stems 512 and/or 518 may comprise press fit stems that are cemented with or without porous body sections 520, straight, fluted, and/or bowed, and in varying diameters.

As disclosed previously, the knee joint 502 comprises a biasing mechanism, which may be a controllable biasing mechanism in a smart embodiment, that biases the joint to the extended position. It is preferred that the biasing force (i.e., the force that tends to extend the knee joint) may be overcome by inertia during the swing phase of the stride. It will be appreciated that too much of an extension biasing force will simulate a fused knee joint. Different extension biasing forces may be provided for different levels of activity. For example, a more active person may require a greater extension biasing force that a more sedentary person.

In one non-limiting exemplary embodiment, stems may be fitted, and a knee joint installed for an active person. As the person (such as a youth) ages, their lifestyle may change, and their contralateral knee may develop a different extension biasing force than their replacement knee. In that case, the modular knee may be exchanged for one that has less biasing force thereby matching the contralateral knee and the recipient's lifestyle.

It is contemplated that certain embodiments of the knee joint 502 may have an adjustable or varying extension biasing force. For example, and not limitation, after implantation, a small tool may be surgically inserted to the knee joint 502 to adjust the biasing force, such as by rotating a screw or other component in the knee joint 502. As another non-limiting example, a non-surgical example may comprise an external device configured to electronically link to a microprocessor or logic circuit in the knee joint 502, such as by magnetic coupling, Bluetooth, or other near field communication protocol, to adjust the bias force or forces, or to adjust operability of the extensor mechanism. For example, a linear motor may be part of the prosthetic knee and a controller in the knee joint may adjust the motor to adjust, e.g., the biasing force, or brake force. Alternately, an adjustable rotary orifice may be controlled or adjusted for hydraulic or pneumatic biasing or braking systems.

While each of these non-surgical examples has benefits, a practicable solution must prevent unexpected or accidental adjustments. For example, an adjustment made through a magnetic coupling must not be susceptible to accidental changes when the subject is exposed to an otherwise imperceptible magnetic field. Similarly, a Bluetooth interface must not be accessible to unauthorized and/or unauthenticated interference.

Common device authentication and authorization mechanisms known to those ordinarily skilled in the art may be applied to data communications interfaces. As an example, a computerized or "smart" joint may be manufactured with a private encryption key and one or more public keys of the manufacturer. It may be configured to only use encrypted communications such that anyone desiring to communicate with the "smart" joint would then need to use a device that has a key signed by the manufacturer.

Along those lines, a magnetically activated adjustment mechanism may be held in a locked state until an authorized and/or authenticated signal has been received through a data communications link. Alternatively, those ordinarily skilled in the art may make use of multiple magnetic fields to lock and unlock a magnetically activated adjustment mechanism. In one non-limiting example, a fixed field of specific strength oriented in one direction may unlock the mechanism while a movable magnet may associate with the mechanism to make adjustments. Removal of the fixed and oriented field may reengage the lock.

Extension biasing components may comprise torsion springs integrated into one or more of the hinges 504, 506. For example, a torsion spring may be fixed to the outer components 522, 524 of knee joint 502 and fixed, such as through splines, to lever component 526. The neutral spring condition may be the extended condition and increasing amounts of flexion create increasing amount of return biasing force. While this may be a linearly increasing amount of return biasing force, Applicants envision embodiments where the return biasing force may be non-linear such that, in one of many non-limiting examples, the return biasing force may reach a maximum at a point mid-way through an arc and remain at that force for the remainder of the arc. In this exemplary embodiment, the return to the extended condition will not be more forceful at increasing arcs. Alternately, extension biasing components may comprise hydraulic, pneumatic, magnetic, or mechanical systems. In yet another envisioned embodiment, combinations of these extension biasing components may be combined. As described, the neutral spring condition with increasing amount of return biasing force from increasing amounts of flexion may be combined with, as a non-limiting example, a magnetic component that negates some of the return biasing force to provide a limited or governed return swing.

The extension biasing components may comprise a brake that releasably "locks" the knee joint in the extended position yet is overcome by the swing forces associated with a normal or active gait.

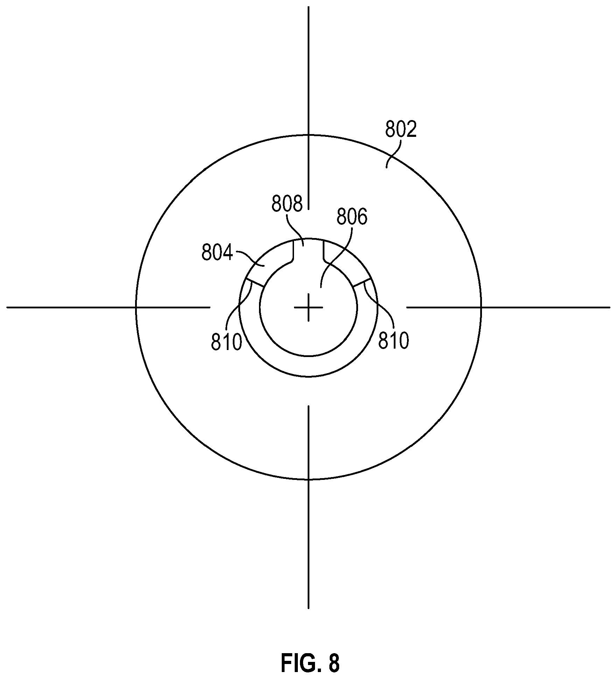

FIG. 8 illustrates one of many embodiments to convert a conventional *ex situ* total knee prosthesis to an *in situ* knee prosthesis with rotation according to the present inventions. A spherical rotation surface 802 may be formed in, for example, the tibial stem or the femoral stem and comprise a central recess 804 into which a protrusion 806 from the knee joint may extend. The protrusion 806 may comprise a lug 808 or alternately a recess (not shown). The recess 804 may comprise two stops 810 that limit the amount of rotation of the knee joint relative to the hip or foot by interacting the lug or recess 808. It is preferred that the load born by the knee joint be reacted by the pad, by the spherical surface or both, but not by the protrusion 806. Persons of skill in the art having benefit of this disclosure will appreciate that other rotation systems may be implemented in conventional *ex situ* knee joints for purposes of these inventions.

In an envisioned embodiment, and without limitation, the stops 810 may be adjoined and rotatable around the central axis of the protrusion 806, such as in a raceway, and biased towards a specific orientation. In this envisioned embodiment, the rotation of the protrusion 806 within rotation surface 802 may be gradually slowed when rotated, rather than meeting a hard and immobile point. That is to say that as protrusion 806 is rotated clockwise in FIG. 8, it will encounter stop 810 on the left. However, rather than stopping the rotation, it may gradually apply a greater resistance to any further clockwise rotation from a bias applied to conjoined stops 810. In this example, conjoined stops 810 may have a limit at which no further rotation is allowed, thus as protrusion 806 is rotated, it is gradually resisted up to a stopping point.

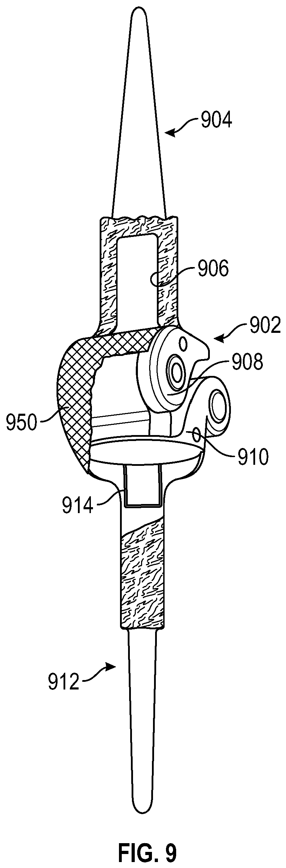

FIG. 9 illustrates an embodiment of the invention in which an *ex situ* knee joint 902 is modified according to the teachings of this disclosure so that it is implantable *in situ* for a total knee replacement in an individual that may have a compromised or non-existent extensor mechanism. In this illustration, the knee joint 902 is polycentric and the femur (not shown) can rotate relative to the tibia (not shown) at the knee joint 902 within a range of motion of about 30 degrees, such as ±15 degrees. Further, FIG. 9 illustrates that the femoral stem 904, which may be integral or modular to knee joint 902, comprises a central channel 906 that may house a biasing element, such as a spring with a linear or non-linear spring rate, a pneumatic piston sealed to the channel walls, and/or a hydraulic piston sealed to the channel walls. Similarly, knee joint components 908 and 910 may comprise magnets that are configured to bias the knee joint 902 into the extend position through the attractive forces. The break over force required to overcome the magnetic attractive or opposing force may be designed to allow flexion during normal activity or any level of future activity.

While the channel 906 is illustrated as associated with the femoral stem 904, it will be appreciated that the channel 906 may be associated with the tibial stem 912, along with the rotation socket 914. Alternately, the rotation socket 914 may be associated with the stem that does not house the channel 906.

Alternately, or additionally, channel 906 may house one or more components of control system 650 as previously discussed.

Prosthesis 402, 502, 602, 902, and other embodiments of the inventions disclosed herein, may comprise, and preferably do comprise, a bio sheath, covering, or encapsulation 950 that separates the moving components of the prosthesis from the surrounding soft tissue and vasculature. For example, and not limitation, suitable bio sheaths may include silicon, silicon oxide, silicon nitride, stainless steel mesh or other biocompatible materials. It will be appreciated that the bio sheath, if implemented, should be flexible or deformable enough to accommodate at least the desired range of flexion and rotation, as well as provide the desired amount of separation, encapsulation or protection of the moving components.

Other and further embodiments utilizing one or more aspects of the inventions described above can be devised without departing from the scope of the invention, which is defined by the claims. Discussion of singular elements can include plural elements and vice-versa.

The order of steps can occur in a variety of sequences unless otherwise specifically limited. The various steps described herein can be combined with other steps, interlineated with the stated steps, and/or split into multiple steps. Similarly, elements have been described functionally and can be embodied as separate components or can be combined into components having multiple functions.

The inventions have been described in the context of preferred and other embodiments and not every embodiment of the invention has been described. Obvious modifications and alterations to the described embodiments are available to those of ordinary skill in the art. The disclosed and undisclosed embodiments are not intended to limit or restrict the scope or applicability of the invention conceived of by the Applicants, but rather, in conformity with the patent laws, Applicants intend to protect fully all such modifications and improvements that come within the scope of the following claims.

## Claims

1. A knee prosthesis (400) configured to be implanted in a human body, the knee prosthesis comprising:
a femoral component (404) comprising a first joint portion and a first intermedullary stem portion (304), the first stem portion configured to be embedded in a femur (102), and the first joint portion configured to replace at least an end of the femur (102);
a tibial component (406) comprising a second joint portion and a second intermedullary stem portion (306), the second stem portion configured to be embedded in a tibia (104) associated with the femur (102);
the first and second joint portions operatively coupled to create a flexion joint having a range of flexion of the tibia (104) relative to the femur (102); and
a prosthetic extensor mechanism operatively coupled to the flexion joint and configured to bias the implanted knee prosthesis (400) to an extended position to simulate a level of natural knee movement for individuals with compromised extensor mechanisms when the knee prothesis (400) is implanted in the human body,
**characterized in that** the extensor mechanism comprises a constant friction joint that relies on constant pressure against a rotating surface to resist knee flexion.

2. The knee prosthesis (400) of claim 1, wherein the extensor mechanism comprises one or more of a spring system; a magnet system; a hydraulic system; or a pneumatic system.

3. The knee prosthesis (400) of claim 1, wherein the flexion joint is configured to also permit a range of rotation of the tibia (104) relative to the femur (102).

4. The knee prosthesis (400) of claim 1, wherein the extensor system comprises a flexion resistance system having at least one friction surface configured to provide an increasing amount of resistance to flexion.

5. The knee prosthesis (400) of claim 3, further comprising a rotation resistance system (810) having at least one friction surface configured provide an increasing amount of resistance to rotation in either direction from a centered orientation.

6. The knee prosthesis (400) of claim 1, wherein the flexion joint is a monocentric axis joint configured to operate as a simple hinge, or a polycentric axis joint that has multiple pivot points.

7. The knee prosthesis (400) of claim 1 in which at least a portion of the flexion joint is encapsulated in a bio sheath (950).

8. The knee prosthesis (400) of claim 1, wherein the femoral stem is removably coupled to the femoral component for purposes of implantation of the knee prosthesis.

9. The knee prosthesis (400) of claim 1, wherein the second intermedullary stem portion (306) is removably coupled to the tibial component (406) for purposes of implantation of the knee prosthesis (400).

10. The knee prosthesis (400) of claim 1, wherein the first intermedullary stem portion (304) is integral with the femoral component (404), the second intermedullary stem portion (306) is integral with the tibial component (406), and the flexion joint is configured to be assembled after implantation of the femoral component (404) and tibial component (406).

11. The knee prosthesis (400) of claim 1, wherein the first intermedullary stem portion (304) is integral with the femoral component (404), the second intermedullary stem portion (306) is integral with the tibial component (406), and the knee prosthesis (400) is configured to be implanted without disassembly of the flexion joint.

12. The knee prosthesis (400) of claim 1, wherein the extensor mechanism comprises a fluid cylinder configured to bias the flexion joint toward 0° flexion.

13. The knee prosthesis (400) of claim 12, wherein the fluid is liquid or gas.

14. The knee prosthesis (400) of claim 13, wherein the extensor mechanism is configured to control the rate of flexion.

15. The knee prosthesis (400) of claim 1, wherein the extensor mechanism comprises a locking joint configured to simulate a fused knee joint.

16. The knee prosthesis (400) of claim 1, wherein the flexion joint comprises a weight-activated stance-control joint.

17. The knee prosthesis (400) of claim 1, wherein the second joint portion is configured to replace at least a top of the tibia (104), and wherein the knee prosthesis (400) further comprises a bio sheath (950) encapsulating the flexion joint configured to separate moving portions of the flexion joint from surrounding tissue.

18. The knee prosthesis (400) of claim 17, wherein the flexion joint is a monocentric axis joint configured to operate as a simple hinge, or a polycentric axis joint that has multiple pivot points.

19. The knee prosthesis (400) of claim 18, wherein either or both of the first intermedullary stem portion (304) and the second intermedullary stem portion (306) are removably coupled to the femoral and tibial component (404, 406) for purposes of implantation of the knee prosthesis (400).

## Patentansprüche

1. Knieprothese (400), die dafür konfiguriert ist, in einen menschlichen Körper implantiert zu werden, wobei die Knieprothese umfasst:
eine Femurkomponente (404), umfassend einen ersten Gelenkabschnitt und einen ersten intramedullären Schaftabschnitt (304), wobei der erste Schaftabschnitt dafür konfiguriert ist, in einen Femur (102) eingebettet zu werden, und der erste Gelenkabschnitt dafür konfiguriert ist, mindestens ein Ende des Femurs (102) zu ersetzen;
eine Tibiakomponente (406), umfassend einen zweiten Gelenkabschnitt und einen zweiten intramedullären Schaftabschnitt (306), wobei der zweite Schaftabschnitt dafür konfiguriert ist, in eine dem Femur (102) zugeordnete Tibia (104) eingebettet zu werden;
wobei der erste und der zweite Gelenkabschnitt operativ gekoppelt sind, um ein Flexionsgelenk zu bilden, das einen Flexionsbereich der Tibia (104) relativ zum Femur (102) aufweist; und
einen prothetischen Streckmechanismus, der operativ mit dem Flexionsgelenk gekoppelt und dafür konfiguriert ist, die implantierte Knieprothese (400) in eine gestreckte Position vorzuspannen, um ein Maß an natürlicher Kniebewegung für Personen mit beeinträchtigten Streckmechanismen zu simulieren, wenn die Knieprothese (400) in den menschlichen Körper implantiert ist,
**dadurch gekennzeichnet, dass** der Streckmechanismus ein Gelenk mit konstanter Reibung umfasst, das auf konstantem Druck gegen eine rotierende Oberfläche beruht, um der Knieflexion entgegenzuwirken.

2. Knieprothese (400) nach Anspruch 1, wobei der Streckmechanismus eines oder mehrere von einem Federsystem; einem Magnetsystem; einem Hydrauliksystem; oder einem Pneumatiksystem umfasst.

3. Knieprothese (400) nach Anspruch 1, wobei das Flexionsgelenk dafür konfiguriert ist, auch einen Rotationsbereich der Tibia (104) relativ zum Femur (102) zu ermöglichen.

4. Knieprothese (400) nach Anspruch 1, wobei das Strecksystem ein Flexionswiderstandssystem mit mindestens einer Reibfläche umfasst, die dafür konfiguriert ist, einen zunehmenden Widerstand gegen Flexion bereitzustellen.

5. Knieprothese (400) nach Anspruch 3, ferner umfassend ein Rotationswiderstandssystem (810) mit mindestens einer Reibfläche, die dafür konfiguriert ist, einen zunehmenden Widerstand gegen Rotation in jeder Richtung von einer zentrierten Ausrichtung aus bereitzustellen.

6. Knieprothese (400) nach Anspruch 1, wobei das Flexionsgelenk ein monozentrisches Achsgelenk ist, das dafür konfiguriert ist, als einfaches Scharnier zu fungieren, oder ein polyzentrisches Achsgelenk, das mehrere Drehpunkte aufweist.

7. Knieprothese (400) nach Anspruch 1, bei der mindestens ein Teil des Flexionsgelenks in einer Bio-Hülle (950) eingekapselt ist.

8. Knieprothese (400) nach Anspruch 1, wobei der Femurschaft zum Zwecke der Implantation der Knieprothese lösbar mit der Femurkomponente gekoppelt ist.

9. Knieprothese (400) nach Anspruch 1, wobei der zweite intramedulläre Schaftabschnitt (306) zum Zwecke der Implantation der Knieprothese (400) lösbar mit der Tibiakomponente (406) gekoppelt ist.

10. Knieprothese (400) nach Anspruch 1, wobei der erste intramedulläre Schaftabschnitt (304) einstückig mit der Femurkomponente (404) ausgebildet ist, der zweite intramedulläre Schaftabschnitt (306) einstückig mit der Tibiakomponente (406) ausgebildet ist und das Flexionsgelenk dafür konfiguriert ist, nach der Implantation der Femurkomponente (404) und der Tibiakomponente (406) zusammengebaut zu werden.

11. Knieprothese (400) nach Anspruch 1, wobei der erste intramedulläre Schaftabschnitt (304) einstückig mit der Femurkomponente (404) ausgebildet ist, der zweite intramedulläre Schaftabschnitt (306) einstückig mit der Tibiakomponente (406) ausgebildet ist und die Knieprothese (400) dafür konfiguriert ist, ohne Demontage des Flexionsgelenks implantiert zu werden.

12. Knieprothese (400) nach Anspruch 1, wobei der Streckmechanismus einen Flüssigkeitszylinder umfasst, der dafür konfiguriert ist, das Flexionsgelenk in Richtung einer 0°-Flexion vorzuspannen.

13. Knieprothese (400) nach Anspruch 12, wobei die Flüssigkeit flüssig oder gasförmig ist.

14. Knieprothese (400) nach Anspruch 13, wobei der Streckmechanismus dafür konfiguriert ist, die Flexionsrate zu steuern.

15. Knieprothese (400) nach Anspruch 1, wobei der Streckmechanismus ein Verriegelungsgelenk umfasst, das dafür konfiguriert ist, ein versteiftes Kniegelenk zu simulieren.

16. Knieprothese (400) nach Anspruch 1, wobei das Flexionsgelenk ein gewichtsaktiviertes Standphasen-Steuerungsgelenk umfasst.

17. Knieprothese (400) nach Anspruch 1, wobei der zweite Gelenkabschnitt dafür konfiguriert ist, mindestens eine Oberseite der Tibia (104) zu ersetzen, und wobei die Knieprothese (400) ferner eine Bio-Hülle (950) umfasst, die das Flexionsgelenk einkapselt und dafür konfiguriert ist, bewegliche Teile des Flexionsgelenks vom umgebenden Gewebe zu trennen.

18. Knieprothese (400) nach Anspruch 17, wobei das Flexionsgelenk ein monozentrisches Achsgelenk ist, das dafür konfiguriert ist, als einfaches Scharnier zu fungieren, oder ein polyzentrisches Achsgelenk, das mehrere Drehpunkte aufweist.

19. Knieprothese (400) nach Anspruch 18, wobei einer oder beide des ersten intramedullären Schaftabschnitts (304) und des zweiten intramedullären Schaftabschnitts (306) zum Zwecke der Implantation der Knieprothese (400) lösbar mit der Femur- und Tibiakomponente (404, 406) gekoppelt sind.

## Revendications

1. Prothèse de genou (400) configurée pour être implantée dans un corps humain, la prothèse de genou comprenant :
un composant fémoral (404) comprenant une première partie d'articulation et une première partie de tige intramédullaire (304), la première partie de tige étant configurée pour être encastrée dans un fémur (102), et la première partie d'articulation étant configurée pour remplacer au moins une extrémité du fémur (102) ;
un composant tibial (406) comprenant une seconde partie d'articulation et une seconde partie de tige intramédullaire (306), la seconde partie de tige étant configurée pour être encastrée dans un tibia (104) associé au fémur (102) ;
les première et seconde parties d'articulation étant couplées de manière opérationnelle pour créer une articulation de flexion ayant une plage de flexion du tibia (104) par rapport au fémur (102) ; et
un mécanisme extenseur prothétique couplé de manière opérationnelle à l'articulation de flexion et configuré pour solliciter la prothèse de genou implantée (400) vers une position étendue afin de simuler un niveau de mouvement naturel du genou pour les individus ayant des mécanismes extenseurs compromis lorsque la prothèse de genou (400) est implantée dans le corps humain,
**caractérisée en ce que** le mécanisme extenseur comprend une articulation à friction constante qui repose sur une pression constante contre une surface rotative pour résister à la flexion du genou.

2. Prothèse de genou (400) selon la revendication 1, dans laquelle le mécanisme extenseur comprend un ou plusieurs parmi un système de ressort ; un système d'aimant ; un système hydraulique ; ou un système pneumatique.

3. Prothèse de genou (400) selon la revendication 1, dans laquelle l'articulation de flexion est configurée pour permettre également une plage de rotation du tibia (104) par rapport au fémur (102).

4. Prothèse de genou (400) selon la revendication 1, dans laquelle le système extenseur comprend un système de résistance à la flexion ayant au moins une surface de friction configurée pour fournir une quantité croissante de résistance à la flexion.

5. Prothèse de genou (400) selon la revendication 3, comprenant en outre un système de résistance à la rotation (810) ayant au moins une surface de friction configurée pour fournir une quantité croissante de résistance à la rotation dans l'une ou l'autre direction à partir d'une orientation centrée.

6. Prothèse de genou (400) selon la revendication 1, dans laquelle l'articulation de flexion est une articulation à axe monocentrique configurée pour fonctionner comme une simple charnière, ou une articulation à axe polycentrique qui présente plusieurs points de pivotement.

7. Prothèse de genou (400) selon la revendication 1, dans laquelle au moins une partie de l'articulation de flexion est encapsulée dans une gaine biologique (950).

8. Prothèse de genou (400) selon la revendication 1, dans laquelle la tige fémorale est couplée de manière amovible au composant fémoral aux fins d'implantation de la prothèse de genou.

9. Prothèse de genou (400) selon la revendication 1, dans laquelle la seconde partie de tige intramédullaire (306) est couplée de manière amovible au composant tibial (406) aux fins d'implantation de la prothèse de genou (400).

10. Prothèse de genou (400) selon la revendication 1, dans laquelle la première partie de tige intramédullaire (304) est solidaire du composant fémoral (404), la seconde partie de tige intramédullaire (306) est solidaire du composant tibial (406), et l'articulation de flexion est configurée pour être assemblée après l'implantation du composant fémoral (404) et du composant tibial (406).

11. Prothèse de genou (400) selon la revendication 1, dans laquelle la première partie de tige intramédullaire (304) est solidaire du composant fémoral (404), la seconde partie de tige intramédullaire (306) est solidaire du composant tibial (406), et la prothèse de genou (400) est configurée pour être implantée sans désassemblage de l'articulation de flexion.

12. Prothèse de genou (400) selon la revendication 1, dans laquelle le mécanisme extenseur comprend un cylindre à fluide configuré pour solliciter l'articulation de flexion vers une flexion de 0°.

13. Prothèse de genou (400) selon la revendication 12, dans laquelle le fluide est un liquide ou un gaz.

14. Prothèse de genou (400) selon la revendication 13, dans laquelle le mécanisme extenseur est configuré pour contrôler la vitesse de flexion.

15. Prothèse de genou (400) selon la revendication 1, dans laquelle le mécanisme extenseur comprend une articulation de verrouillage configurée pour simuler une articulation du genou fusionnée.

16. Prothèse de genou (400) selon la revendication 1, dans laquelle l'articulation de flexion comprend une articulation de contrôle de phase d'appui activée par le poids.

17. Prothèse de genou (400) selon la revendication 1, dans laquelle la seconde partie d'articulation est configurée pour remplacer au moins une partie supérieure du tibia (104), et dans laquelle la prothèse de genou (400) comprend en outre une gaine biologique (950) encapsulant l'articulation de flexion, configurée pour séparer les parties mobiles de l'articulation de flexion des tissus environnants.

18. Prothèse de genou (400) selon la revendication 17, dans laquelle l'articulation de flexion est une articulation à axe monocentrique configurée pour fonctionner comme une simple charnière, ou une articulation à axe polycentrique qui présente plusieurs points de pivotement.

19. Prothèse de genou (400) selon la revendication 18, dans laquelle l'une ou l'autre ou les deux parmi la première partie de tige intramédullaire (304) et la seconde partie de tige intramédullaire (306) sont couplées de manière amovible au composant fémoral et tibial (404, 406) aux fins d'implantation de la prothèse de genou (400).
